(19) Europäisches Patentamt
European Patent Office
Office européen des brevets

(11) **EP 4 160 172 A1**

(12) **EUROPEAN PATENT APPLICATION**

(43) Date of publication:
**05.04.2023 Bulletin 2023/14**

(21) Application number: **22199356.1**

(22) Date of filing: **03.10.2022**

(51) International Patent Classification (IPC):
**G01L 5/00** (2006.01)    **A61B 5/11** (2006.01)
**A63B 69/00** (2006.01)    **A63B 71/08** (2006.01)
**G01P 15/08** (2006.01)    **G01P 15/18** (2006.01)

(52) Cooperative Patent Classification (CPC):
**G01L 5/0052; A61B 5/1114; A61B 5/1121;**
**A61B 5/4542; A61B 5/6803; A61B 5/7264;**
**A61B 5/7275; A61B 5/743; A61B 5/744;**
**G01P 15/08; G01P 15/18;** A61B 2505/01;
A61B 2562/0219; A63B 71/085; A63B 2220/40;

(Cont.)

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**KH MA MD TN**

(30) Priority: **01.10.2021 AU 2021903145**

(71) Applicant: **HitIQ Limited**
**South Melbourne, Victoria 3205 (AU)**

(72) Inventors:
• **Erikson, David**
**South Melbourne, 3205 (AU)**
• **Cuevas, Luis**
**South Melbourne, 3205 (AU)**
• **Vegar, Mike**
**South Melbourne, 3205 (AU)**

(74) Representative: **Doherty, William et al**
**Albright IP Limited**
**County House**
**Bayshill Road**
**Cheltenham, Glos. GL50 3BA (GB)**

(54) **PREDICTION OF HEAD IMPACT EVENT MECHANISM VIA INSTRUMENTED MOUTHGUARD DEVICES**

(57)    The present invention relates, in various embodiments, to analysis of head impacts using instrumented technology, such as instrumented mouthguard devices. Some embodiments are directed to prediction of head impact event mechanism, for example in terms of a location and/or direction of an impact incurred by the head. While some embodiments will be described herein with particular reference to that application, it will be appreciated that the invention is not limited to such a field of use, and is applicable in broader contexts.

**FIG. 1**

EP 4 160 172 A1

(52) Cooperative Patent Classification (CPC): (Cont.)
 A63B 2220/44; A63B 2220/53

**Description**

**FIELD OF THE INVENTION**

The present invention relates, in various embodiments, to analysis of head impacts using instrumented technology, such as instrumented mouthguard devices. Some embodiments are directed to prediction of head impact event mechanism, for example in terms of a location and/or direction of an impact incurred by the head. While some embodiments will be described herein with particular reference to that application, it will be appreciated that the invention is not limited to such a field of use, and is applicable in broader contexts.

**BACKGROUND**

Any discussion of the background art throughout the specification should in no way be considered as an admission that such art is widely known or forms part of common general knowledge in the field.

Brain injuries, particularly those sustained during participation in contact sports, are becoming an increasingly important focus of attention. For example, head impacts (and other upper body impacts) sustained during sport can have serious effects of both short term and long-term participant welfare. For example, it is valuable to better understand the nature of a suspected brain injury in terms of: (i) whether a participant should be rested from participation; (ii) an extent to which the injury should prevent a return to activity; (iii) a degree of seriousness of an injury, for instance insofar as that might affect treatment and management; and (iv) better understanding cumulative effects of successive brain injuries for a given participant.

One strategy for evaluating player impact loads as part of an injury prevention program is the use of instrumented technology. However, the implementation in the field has been limited by the reliability and validity of such technology. Instrumented technology is useful for understanding accelerations associated with impacts, but it is challenging to gain a more comprehensive understanding of the mechanism of injury.

**SUMMARY OF THE INVENTION**

It is an object of the present invention to overcome or ameliorate at least one of the disadvantages of the prior art, or to provide a useful alternative.

One embodiment provides a method for prediction of head impact event mechanism via instrumented mouthguard devices, the method including:

receiving, as input, time series data representative of a head impact event, wherein the time series data is derived from an instrumented mouthguard device including one or more accelerometers;

generating an array of spatial coordinates, the spatial coordinates representing points on a computer head model;

processing the time series data thereby to determine a direction of impact and location of impact relative to the computer head model.

One embodiment provides a method including:

defining a fixed system of reference for the head model;

identifying one or more key points-in-time in the time series data;

for each of the one or more key points-in-time in the time series data:

calculating direction angles for acceleration at that key point-in-time relative to the fixed system of reference;

determining orientation of the head model based on values of the calculated direction angles for acceleration.

One embodiment provides a method wherein there are at least two key points in time, the method further including generating an animation of head model movement between the determined orientation between the at least two key points in time thereby to generate an animation which reconstructs head orientation changes during the impact event.

One embodiment provides a method wherein generating the animation includes incorporating data derived from the determine a direction of impact and location of impact relative to the head model.

One embodiment provides a method wherein the direction angles are calculated for a unit vector derived from the time series data at the relevant key point-in-time.

One embodiment provides a method wherein processing the time series data thereby to determine a direction of impact and location of impact relative to the head model includes mathematically constructing a shape enclosing the head model, and performing processing thereby to determine an intersection between the shape and an action line of force derived from the time series data.

One embodiment provides a method wherein processing the time series data thereby to determine a direction of impact and location of impact relative to the head model includes:

identifying a data set including measurements of linear and rotational acceleration of the head during the impact event;

processing the linear acceleration thereby to predict an impact direction for the impact event;

processing the dataset thereby to calculate a predicted moment arm associated with rotational movement described by the measurements of linear and angular accelerations;

processing, in combination: (i) the predicted direction of the impact; and (ii) the predicted moment arm, thereby to determine a predicted impact location for the impact event.

One embodiment provides a method wherein the step of processing the linear and angular accelerations to calculate a moment arm required to produce a rotational movement described by the linear and angular accelerations is based on performed based on an assumption that the axis of rotation passes through the centre of gravity of the head.

One embodiment provides a method including generating a graphic based on the predicted impact location for the impact event, wherein the graphic includes a representation of a human head, and an element graphically representing direction and location of impact.

One embodiment provides a method including:

(i) rotating values for linear and angular accelerations to match a predefined system of reference;

(ii) generating an array with spatial coordinates of a number of points on the surface of the head model;

(iii) using the coordinates of each point on the surface of the head model to generate vectors with origin at a tip of a moment arm vector;

(iv) calculating unit vectors for each vector generated at (iii);

(v) mathematically constructing a shape with centre at the centre of gravity of the head model;

(vi) defining two positions along a line of action of the force, being the tip of the momentum arm and the tip of the vector resulting from the sum of the moment arm and the linear acceleration vector;

(vii) using those two positions to generate the parametrized equations of the line of action of the force;

(viii) combining a shape equation for the shape and parametric equations for the line of action of the impact, thereby to calculate one or more intersections of the shape and the line of action, and thereby determine a point at which the impact enters the mathematically constructed shape;

(ix) constructing a vector from the tip of the moment arm to the point at which the impact enters the mathematically constructed shape, and normalizing that vector;

(x) identifying the combination of unit vector calculated at (iv) and vector calculated at (ix) having the smallest angle therebetween, and identifying an associated spatial coordinate on the head model for that unit vector, thereby to predict the spatial coordinate on the head model at which the impact occurs.

One embodiment provides a method for prediction of head impact event mechanism, the method including:

identifying a data set including measurements of linear and rotational acceleration of a head during an impact event;

processing the linear acceleration thereby to predict an impact direction for the impact event;

processing the dataset thereby to calculate a predicted moment arm associated with rotational movement described by the measurements of linear and angular accelerations;

processing, in combination: (i) the predicted direction of the impact; and (ii) the predicted moment arm, thereby to determine a predicted impact location for the impact event.

One embodiment provides a method wherein the step of processing the linear and angular accelerations to calculate a moment arm required to produce a rotational movement described by the linear and angular accelerations is based on performed based on an assumption that the axis of rotation passes through the centre of gravity of the head.

One embodiment provides a method including generating a graphic based on the predicted impact location for the impact event, wherein the graphic includes a representation of a human head, and an element graphically representing direction and location of impact.

One embodiment provides a method additionally including estimation of a head orientation relative to a defined system of reference at one or more points in time during the impact event.

One embodiment provides a method wherein the estimation of a head orientation relative to the defined system of reference are used thereby to provide an animation of changes in head orientation during the impact event.

One embodiment provides a method wherein the animation is additionally based on the predicted direction and/or location of impact.

One embodiment provides a method wherein the data set is derived from an instrumented mouthguard device.

One embodiment provides a method wherein the data set including measurements of linear and rotational acceleration of a head during an impact event is derived from processing of data derived from a plurality of body-worn accelerometers.

One embodiment provides a method including:

(i) rotating values for linear and angular accelerations to match a predefined system of reference;

(ii) generating an array with spatial coordinates of a number of points on the surface of the head model;

(iii) using the coordinates of each point on the surface of the head model to generate vectors with origin at a tip of a moment arm vector;

(iv) calculating unit vectors for each vector generated at (iii);

(v) mathematically constructing a shape with centre at the centre of gravity of the head model;

(vi) defining two positions along a line of action of the force, being the tip of the momentum arm and the tip of the vector resulting from the sum of the moment arm and the linear acceleration vector;

(vii) using those two positions to generate the parametrized equations of the line of action of the force;

(viii) combining a shape equation for the shape and parametric equations for the line of action of the impact, thereby to calculate one or more intersections of the shape and the line of action, and thereby determine a point at which the impact enters the mathematically constructed shape;

(ix) constructing a vector from the tip of the moment arm to the point at which the impact enters the mathematically constructed shape, and normalizing that vector;

(x) identifying the combination of unit vector calculated at (iv) and vector calculated at (ix) having the smallest angle therebetween, and identifying an associated spatial coordinate on the head model for that unit vector, thereby to predict the spatial coordinate on the head model at which the impact occurs.

Further embodiments include systems configured to perform any of the methods discussed above, or elsewhere herein (for example computer systems).

Further example embodiments are described below in the section entitled "claims".

Reference throughout this specification to "one embodiment", "some embodiments" or "an embodiment" means that a particular feature, structure or characteristic described in connection with the embodiment is included in at least one embodiment of the present invention. Thus, appearances of the phrases "in one embodiment", "in some embodiments" or "in an embodiment" in various places throughout this specification are not necessarily all referring to the same embodiment, but may. Furthermore, the particular features, structures or characteristics may be combined in any suitable manner, as would be apparent to one of ordinary skill in the art from this disclosure, in one or more embodiments.

As used herein, unless otherwise specified the use of the ordinal adjectives "first", "second", "third", etc., to describe a common object, merely indicate that different instances of like objects are being referred to, and are not intended to imply that the objects so described must be in a given sequence, either temporally, spatially, in ranking, or in any other manner.

In the claims below and the description herein, any one of the terms comprising, comprised of or which comprises is an open term that means including at least the elements/features that follow, but not excluding others. Thus, the term comprising, when used in the claims, should not be interpreted as being limitative to the means or elements or steps listed thereafter. For example, the scope of the expression a device comprising A and B should not be limited to devices consisting only of elements A and B. Any one of the terms including or which includes or that includes as used herein is also an open term that also means including at least the elements/features that follow the term, but not excluding others. Thus, including is synonymous with and means comprising.

As used herein, the term "exemplary" is used in the sense of providing examples, as opposed to indicating quality. That is, an "exemplary embodiment" is an embodiment provided as an example, as opposed to necessarily being an embodiment of exemplary quality.

## BRIEF DESCRIPTION OF THE DRAWINGS

Embodiments of the invention will now be described, by way of example only, with reference to the accompanying drawings in which:

FIG. 1 illustrates a relationship between a moment arm, a force, and a torque.

FIG. 2 illustrates an example head coordinate system for impact location reporting.

## DETAILED DESCRIPTION

The present invention relates, in various embodiments, to analysis of head impacts using instrumented technology, such as instrumented mouthguard devices. Some embodiments are directed to prediction of head impact event mechanism, for example in terms of a location and/or direction of an impact incurred by the head. While some embodiments will be described herein with particular reference to that application, it will be appreciated that the invention is not limited to such a field of use, and is applicable in broader contexts.

*Overview*

Described herein is an example Impact Location Algorithm which prediction (i.e. estimation) of head impact event mechanism, for example in terms of a location and/or direction of an impact incurred by the head. In overview, the Impact Location Algorithm provides technology to approximate the location of an impact on the surface of the head by using linear and rotational acceleration measurements derived from an instrumented mouthguard device. The linear acceleration is used to the predict the direction of the impact. Then, linear and angular accelerations are used to calculate the moment arm required to produce a rotational movement. This is performed based on an assumption that the axis of rotation passes through the centre of gravity of the head. The direction of the impact and the moment arm are combined by the algorithm to calculate the impact location.

The Impact Location Algorithm is performed in respect of a data set which provides time domain samples of linear and rotational acceleration measurements for an impact event, as observed from the predicted centre of gravity of a subject's head. Measurements are in practice made by one or a set of accelerometers embedded in a mouthguard device, with data from those being processed and translated to the centre of gravity, using techniques known in the art.

Further embodiments include methods which enable determination (i.e. estimation) of head orientation at one or more points in time during an impact, and optionally using that data (combined with impact location and direction) thereby to provide an animation which reconstructs an impact.

A range of instrumented mouthguards may be used, for example a mouthguard as discussed in PCT Patent Application PCT/AU2020/050096.

*Assumptions*

For the purposes of the example Impact Location Algorithm, the following assumptions are made:

(i) The head is assumed to be a free body from the start of the impact event until the linear acceleration measurement reaches its peak - This assumption is justified given that, for the first instants of an impact, the head does not suffer a significant translation movement and the neck reaction is negligible.

(ii) The axis of rotation is assumed to pass through the centre of gravity of the head. This assumption is justified by the first assumption as the rotation of a free body happens around an axis passing through its centre of gravity.

(iii) The axis of rotation does not vary with time. This assumption is needed to ensure a closed solution to the problem; a changing axis of rotation leads to a varying mass moment of inertia.

*Discussion of Torque*

Torque is a turning action on a body about an axis of rotation due to a force F. The torque is given by T = r x F, where x represents the cross-product operator, F is the force and r is the position vector indicating the point where F is applied. Since the torque is obtained through the cross product of F and r, its direction is perpendicular to the plane defined by F and r.

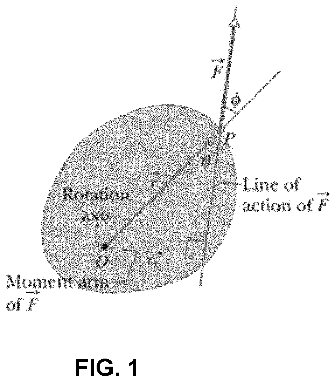

The "moment arm" is defined as the perpendicular vector from the rotation axis to the extended line of action of force (see FIG. 1). Note that the torque can be calculated by using the moment arm. Then, the momentum arm is perpendicular to the plane defined by the torque and force vector. This implies that the moment arm, the force, and the torque are orthogonal. Hence, it is possible to obtain the direction of the momentum arm if based on knowledge of the unit vectors of the force and the torque.

The Newton's second law for rotation implies that a torque produced by a force causes an angular/rotational acceleration around the rotation axis in the same direction as the torque. Therefore, the unit vector defining the instantaneous direction of the torque is the same as the one defining the instantaneous direction of the angular acceleration. Furthermore, from Newton's second law for linear movement, the direction of the force as well as the direction of the linear acceleration are defined by the same unit vector.

From now on, we will use the symbol "r" to refer to the moment arm. By the orthogonality of the moment arm, the force and the torque, the moment arm direction can be calculated via the cross product of the unit vectors of F and T, i.e. $d\_r = u\_F \times u\_T$ where $d\_r$ is the direction vector of the moment arm, and $u\_F$ and $u\_T$ are the unit vectors in the directions of F and T, respectively. Note that the order of the factors is defined by the righthand side rule. Although $d\_r$ gives us the direction of the moment arm vector, we must normalize it (unit form) so that one can get the exact point where the moment arm intersects the force line of action. Therefore, the unit vector defining the direction of the momentum arm is given by

$$u\_r = u\_F \times u\_T \, / \, | \, u\_F \times u\_T \, |.$$

Recall that the unit vectors of force and torque have the same unit vectors as the linear and angular accelerations, respectively. Therefore, we rewrite the above equation as:

$$u\_r = u\_a \times u\_alpha \, / \, | \, u\_a \times u\_alpha \, |,$$

In the above equation, $u\_a$ is a unit vector in the direction of the linear acceleration and $u\_alpha$ is a unit vector in the direction of the angular acceleration.

To calculate the magnitude of the moment arm, we use Newton's second law, which establishes that $T = I \cdot |alpha|$, where I is the mass rotational moment of inertia and alpha is the angular acceleration. The magnitude of the cross product is defined as $|A \times B| = |A||B|\sin(theta)$, where A and B are vectors and theta is the angle between them. Since $T = r \times F$, we have that $I \cdot |alpha| = |r||m \cdot a|$ as r is perpendicular to $F = m \cdot a$ where a is the linear acceleration. Therefore, the magnitude of r is given by

$$|r| = I \cdot |alpha| \, / \, (m \cdot |a|)$$

The unit vector in the direction of r and the magnitude of r fully define the moment arm vector with origin and the centre of gravity of the head. This information combined with the line of action of the force F can be used to estimate the impact location on the surface of the head.

*Implementation - Determining Location and Direction of Impacts*

The main input for the Impact Location Algorithm are (i) the direction of the linear acceleration; (ii) the moment arm vector; and (iii) the spatial coordinates of a number of points on the surface of the head.

In relation to (iii), on the basis that the head has an irregular surface, seeking to determine the impact location without extra tools requires a high computational cost. Therefore, the present approach is to use a sphere with a radius large enough to contain the head, and use analytic geometry tools to reduce computational burdens.

Note that the present example algorithm operates on the basis of a system of reference where "positive x: is pointing towards the face, "positive y" is pointing to the left, and "positive z" is pointing to the head crown.

The implementation of the example algorithm can be summarized as follows:

**Step 1:** rotate values for linear and angular accelerations to match the system of reference used by the algorithm.

**Step 2:** Compute the unit vector and magnitude of the moment arm by using the peak linear and angular accelerations. A constant moment of inertia for the head is assumed.

**Step 3:** Generate an array with all the spatial coordinates of a number of points on the surface of the head. These coordinates are in the present example taken from a head model *obj* file.

**Step 4:** Use the coordinates of each point on the surface of the head to generate vectors with origin at the tip of the moment arm vector.

**Step 5:** Calculate the unit vectors for each vector computed in the previous step.

**Step 6:** Mathematically construct a sphere with centre at the centre of gravity of the head and radius equal to 14cm (other values may be used; 14cm is used as an approximation of a sphere which would contain most human heads). The sphere equation is given by

$$(x - p\_c[0])\^2 + (y - p\_c[1])\^2 + (z - p\_c[2])\^2 = 0.14\^2 \text{ where } p\_c = [0, 0, 0].$$

**Step 7:** Define two positions along the line of action of the force. The first position (p_1) is defined by the tip of the momentum arm. The second position (p_2) is determined by the tip of the vector resulting from the sum of the moment arm and the linear acceleration vector.

**Step 8:** Positions p_1 and p_2 are used to generate the parametrized equations of the line of action of the force. The parametric equations are as follows:

$$x = p\_1[0] + (p\_2[0] - p\_1[0])t,$$

$$y = p\_1[1] + (p\_2[1] - p\_1[1])t,$$

$$z = p\_1[2] + (p\_2[2] - p\_1[2])t.$$

**Step 9:** By combining the sphere equation and the parametric equations of the line of action of the impact, we calculate the intersection of the sphere and the line. From this:

(i) If two real values are obtained, the line of action of the force intersects the sphere twice.

(ii) If one real value is obtained, the line is tangential to the sphere.

(iii) If two complex values with imaginary part different from zero are obtained, the line never intersect the sphere.

**Step 10:** The position p_2 and the intersection points are used to define where the impact 'enters' the sphere. To do so, we take the distance from p_2 to the intersection points. The intersection point that is further away from p_2 is the point where the impact 'enters' the sphere.

Step 11. Construct a vector from the tip of the moment arm to the point calculated in step 10, and normalize it.

Step 12: Calculate the angle between all the unit vectors generated in step 5 and the vector obtained from step 11. Then, we identify the combination having the smallest angle, and the unit vector of that combination..

**Step 13.** Based on the unit vector identified in step 12, determine the coordinate of the location of the impact in the head surface by using the array generated in step 3 (noting that each unit vector is associated with spatial coordinates via steps 4 and 5). Accordingly, the expected coordinate location of the impact has been calculated.

In this manner, the example algorithm is able to plot the head and an arrow indicating the location of the impact. Example steps for generating such a graphical representation are as follows:

(A) By using the *obj* file used in step 3 of the above example algorithm, construct a mesh and plot the head.

(B) Generate a small sphere and put it at the impact location generated by the above example algorithm.

(C) Use an *obj* file of an arrow to further graphically show the impact to the head.

(D) Since the original orientation of the arrow is known, the linear acceleration direction is used to rotate the arrow by using a quaternion rotation.

(E) Locate the tip of the arrow in the same position of the small sphere generated in step 2.

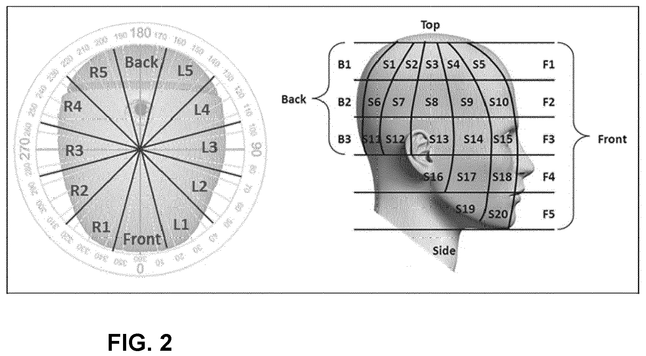

In this manner, the graphical representation is able to display a headform graphic, in conjunction with an arrow (or other element) which represents a direction and location of impact on the headform graphic. This provides a representation of the predicted impact on the relevant subject's head, corresponding to the measured impact event.
In some embodiments additional steps are performed thereby to further improve output data. These are discussed below.
**Step 14.** Define a vector from the centre of gravity of the head to the impact location on the surface of the head (as defined via Step 13).
**Step 15.** Calculate the direction angles of the vector above to estimate the impact region. This is done to provide the user with more meaningful information. The impact regions are optionally defined based on the coordinate system of FIG. 2. The regions are TOP, B1, B2, B3, F1, F2, F3, F4, F5, R/S1, R/S2,..., R/S20, L/S1, L/S2,..., L/S20, where B stands for back, F for front, S for side, R for right and L for left.
The resulting output of the algorithm consists of the impact region, the 3D coordinates of the impact location, the direction angles, and a flag indicating if the impact was (or was not) to the head. Note that the algorithm is able to give the 3D coordinates and direction angles in the system of reference provided in the document (x-positive towards the face, y-positive to the left and z-positive upwards) as well as in the universal system of reference used in the impact processing pipeline (x-positive towards the face, y-positive to the right and z-positive downwards).

*Further Embodiment - Determining Orientation of the Head at Time of Impact*

In some embodiments, a method is performed thereby to determine orientation of the head at the time of impact. This is optionally performed in combination with the method above, thereby to provide a deeper understanding and/or improved reconstruction of an impact. One example method is as follows.
**Step A1.** Define a fixed system of reference outside the head.
**Step A2.** Apply an algorithm thereby to identify time series data associated with the start of the impact (e.g. a determined the start of a peak). For example, this may use a significant peak detection algorithm.
**Step A3.** In the time series data identified in A2, evaluate if there are small peaks prior to the start of the impact caused by up-sampling of underlying signals.
**Step A4.** Find the closest point in time to the start of the impact unaffected by up-sampling of the underlying signals.

**Step A5.** Calculate a unit vector for the identified point in step A4.

**Step A6.** Calculate direction angles using the components of the unit vector. Values different from zero in the x and y directions imply the head's system of reference is rotated with respect to the fixed frame defined in step A1, thereby revealing initial orientation.

In an alternate implementation, following step A2 a DC remover to remove the gravity component from the underlying impact data trace. Then, processing is performed to determine the difference between the original signal and the output of the DC remover for the data prior to the start of the impact. Note that this difference should deliver the gravity vector alongside with some DC offset. This is followed by steps A3-A6 of the method above.

*Further Embodiment - Determining Orientation of the Head at End of Impact*

In some embodiments, a method is performed thereby to determine orientation of the head at end of an impact. This is optionally performed in combination with the method above, thereby to provide a deeper understanding and/or improved reconstruction of an impact. One example method is as follows.

**Step B1.** Define a fixed system of reference outside the head.

**Step B2.** Apply an algorithm thereby to identify time series data associated with the end of the impact (e.g. a determine the end of a peak). For example, this may use a significant peak detection algorithm to identify the impact.

**Step B3.** In the time series data identified in B2, evaluate if there are small peaks prior to the end of the impact caused by up-sampling of underlying signals.

**Step B4.** Find the closest point in time to the end of the impact unaffected by up-sampling of the underlying signals.

**Step B5.** Calculate a unit vector for the identified point in step B4.

**Step B6.** Calculate direction angles using the components of the unit vector. Values different from zero in the x and y directions imply the head's system of reference is rotated with respect to the fixed frame defined in step B1, thereby revealing end-of-impact orientation.

Again, an alternate implementation, following step B2 a DC remover to remove the gravity component from the underlying impact data trace. Then, processing is performed to determine the difference between the original signal and the output of the DC remover for the data prior to the start of the impact. Note that this difference should deliver the gravity vector alongside with some DC offset. This is followed by steps B3-B6 of the method above.

*Further Embodiments - Animation of Head Motion based on Observed Impact*

It will be appreciated that the methods above may be applied thereby to determine head orientation at various stages thorough an observed impact event. For example, the above methods show how it is possible to determine orientation at the start and end of an impact. This may equally be applied to various other points within in an impact (observed through time series data, for example as derived from accelerometers and/or gyroscopes). This may be used thereby to animate a reconstruction of an impact.

A method according to one embodiment is described below.

**Step C1.** Estimate the location of the impact. For example, this is performed based on an impact location estimation method described further above.

**Step C2.** Estimate the orientation of the head at the start of the impact, at the end of the impact, and preferably additionally at the peak of the impact. This is performed using orientation estimates described above.

**Step C3.** Verify the validity of the results in steps C1 and C2 by using the impact direction angles computed by the impact location algorithm validated against the direction angles computed in step C2.

**Step C4.** If the impact location and orientations are valid, create three trajectories for orientation using cubic approximations (one trajectory per direction angle).

**Step C4.** Animate the motion of the head. This may be performed using various known computer animation technologies.

The above method may be expanded by incorporating a larger number of intermediate points, and in respect of each of those estimating orientation, thereby to provide additional samples for improved accuracy in animation.

*Conclusions and Interpretation*

The disclosure above provides improved technology for analysis of head impacts using instrumented technology, such as instrumented mouthguard devices. In particular, the technology allows for prediction of head impact event mechanism, for example in terms of a location and/or direction of an impact incurred by the head.

The terminology used herein is for the purpose of describing particular embodiments only and is not intended to be limiting of the invention. As used herein, the singular forms "a", "an" and "the" are intended to include the plural forms as well, unless the context clearly indicates otherwise. It will be further understood that the terms "comprises" and/or "comprising," when used in this specification, specify the presence of stated features, integers, steps, operations, elements, and/or components, but do not preclude the presence or addition of one or more other features, integers, steps, operations, elements, components, and/or groups thereof.

The corresponding structures, materials, acts, and equivalents of all means or step plus function elements, if any, in the claims below are intended to include any structure, material, or act for performing the function in combination with other claimed elements as specifically claimed. The description of the present invention has been presented for purposes of illustration and description, but is not intended to be exhaustive or limited to the invention in the form disclosed. Many modifications and variations will be apparent to those of ordinary skill in the art without departing from the scope and spirit of the invention. The embodiment was chosen and described in order to best explain the principles of the invention and the practical application, and to enable others of ordinary skill in the art to understand the invention for various embodiments with various modifications as are suited to the particular use contemplated.

Various aspects of the present disclosure may be embodied as a program, software, or computer instructions embodied in a computer or machine usable or readable medium, which causes the computer or machine to perform the steps of the method when executed on the computer, processor, and/or machine. A program storage device readable by a machine, tangibly embodying a program of instructions executable by the machine to perform various functionalities and methods described in the present disclosure is also provided.

A system and method of the present disclosure may be implemented and run on a general-purpose computer or special-purpose computer system. The terms "computer system" and "computer network" as may be used in the present application may include a variety of combinations of fixed and/or portable computer hardware, software, peripherals, and storage devices. The computer system may include a plurality of individual components that are networked or otherwise linked to perform collaboratively, or may include one or more stand-alone components. The hardware and software components of the computer system of the present application may include and may be included within fixed and portable devices such as desktop, laptop, and/or server. A module may be a component of a device, software, program, or system that implements some "functionality", which can be embodied as software, hardware, firmware, electronic circuitry, or etc.

Although specific embodiments of the present invention have been described, it will be understood by those of skill in the art that there are other embodiments that are equivalent to the described embodiments. Accordingly, it is to be understood that the invention is not to be limited by the specific illustrated embodiments, but only by the scope of the appended claims.

It should be appreciated that in the above description of exemplary embodiments of the invention, various features of the invention are sometimes grouped together in a single embodiment, FIG., or description thereof for the purpose of streamlining the disclosure and aiding in the understanding of one or more of the various inventive aspects. This method of disclosure, however, is not to be interpreted as reflecting an intention that the claimed invention requires more features than are expressly recited in each claim. Rather, as the following claims reflect, inventive aspects lie in less than all features of a single foregoing disclosed embodiment. Thus, the claims following the Detailed Description are hereby expressly incorporated into this Detailed Description, with each claim standing on its own as a separate embodiment of this invention.

Furthermore, while some embodiments described herein include some but not other features included in other embodiments, combinations of features of different embodiments are meant to be within the scope of the invention, and form different embodiments, as would be understood by those skilled in the art. For example, in the following claims, any of the claimed embodiments can be used in any combination.

Furthermore, some of the embodiments are described herein as a method or combination of elements of a method that can be implemented by a processor of a computer system or by other means of carrying out the function. Thus, a processor with the necessary instructions for carrying out such a method or element of a method forms a means for carrying out the method or element of a method. Furthermore, an element described herein of an apparatus embodiment is an example of a means for carrying out the function performed by the element for the purpose of carrying out the invention.

In the description provided herein, numerous specific details are set forth. However, it is understood that embodiments of the invention may be practiced without these specific details. In other instances, well-known methods, structures and techniques have not been shown in detail in order not to obscure an understanding of this description.

Similarly, it is to be noticed that the term coupled, when used in the claims, should not be interpreted as being limited to direct connections only. The terms "coupled" and "connected," along with their derivatives, may be used. It should be understood that these terms are not intended as synonyms for each other. Thus, the scope of the expression a device A coupled to a device B should not be limited to devices or systems wherein an output of device A is directly connected to an input of device B. It means that there exists a path between an output of A and an input of B which may be a path including other devices or means. "Coupled" may mean that two or more elements are either in direct physical or electrical contact, or that two or more elements are not in direct contact with each other but yet still co-operate or interact with each other.

Thus, while there has been described what are believed to be the preferred embodiments of the invention, those skilled in the art will recognize that other and further modifications may be made thereto without departing from the spirit of the invention, and it is intended to claim all such changes and modifications as falling within the scope of the invention. For example, any formulas given above are merely representative of procedures that may be used. Functionality may be added or deleted from the block diagrams and operations may be interchanged among functional blocks. Steps may be added or deleted to methods described within the scope of the present invention.

**Claims**

1. A method for prediction of head impact event mechanism via instrumented mouthguard devices, the method including:

   receiving, as input, time series data representative of a head impact event, wherein the time series data is derived from an instrumented mouthguard device including one or more accelerometers;
   generating an array of spatial coordinates, the spatial coordinates representing points on a computer head model;
   processing the time series data thereby to determine a direction of impact and location of impact relative to the computer head model.

2. A method according to claim 1 further including:

   defining a fixed system of reference for the head model;
   identifying one or more key points-in-time in the time series data;
   for each of the one or more key points-in-time in the time series data:

      calculating direction angles for acceleration at that key point-in-time relative to the fixed system of reference;
      determining orientation of the head model based on values of the calculated direction angles for acceleration.

3. A method according to claim 2 wherein there are at least two key points in time, the method further including generating an animation of head model movement between the determined orientation between the at least two key points in time thereby to generate an animation which reconstructs head orientation changes during the impact event.

4. A method according to claim 3 wherein generating the animation includes incorporating data derived from the determine a direction of impact and location of impact relative to the head model.

5. A method according to claim 2 or claim 3 wherein the direction angles are calculated for a unit vector derived from the time series data at the relevant key point-in-time.

6. A method according to any preceding claim wherein processing the time series data thereby to determine a direction of impact and location of impact relative to the head model includes mathematically constructing a shape enclosing the head model, and performing processing thereby to determine an intersection between the shape and an action line of force derived from the time series data.

7. A method according to any preceding claim wherein processing the time series data thereby to determine a direction of impact and location of impact relative to the head model includes:

   identifying a data set including measurements of linear and rotational acceleration of the head during the impact event;
   processing the linear acceleration thereby to predict an impact direction for the impact event;
   processing the dataset thereby to calculate a predicted moment arm associated with rotational movement described by the measurements of linear and angular accelerations;
   processing, in combination: (i) the predicted direction of the impact; and (ii) the predicted moment arm, thereby to determine a predicted impact location for the impact event.

8. A method according to claim 7 wherein the step of processing the linear and angular accelerations to calculate a moment arm required to produce a rotational movement described by the linear and angular accelerations is based on performed based on an assumption that the axis of rotation passes through the centre of gravity of the head.

9. A method according to claim 7 or claim 8 including generating a graphic based on the predicted impact location for the impact event, wherein the graphic includes a representation of a human head, and an element graphically representing direction and location of impact.

10. A method according to any preceding claim including:

    (i) rotating values for linear and angular accelerations to match a predefined system of reference;
    (ii) generating an array with spatial coordinates of a number of points on the surface of the head model;
    (iii) using the coordinates of each point on the surface of the head model to generate vectors with origin at a tip of a moment arm vector;
    (iv) calculating unit vectors for each vector generated at (iii);
    (v) mathematically constructing a shape with centre at the centre of gravity of the head model;
    (vi) defining two positions along a line of action of the force, being the tip of the momentum arm and the tip of the vector resulting from the sum of the moment arm and the linear acceleration vector;
    (vii) using those two positions to generate the parametrized equations of the line of action of the force;
    (viii) combining a shape equation for the shape and parametric equations for the line of action of the impact, thereby to calculate one or more intersections of the shape and the line of action, and thereby determine a point at which the impact enters the mathematically constructed shape;
    (ix) constructing a vector from the tip of the moment arm to the point at which the impact enters the mathematically constructed shape, and normalizing that vector;
    (x) identifying the combination of unit vector calculated at (iv) and vector calculated at (ix) having the smallest angle therebetween, and identifying an associated spatial coordinate on the head model for that unit vector, thereby to predict the spatial coordinate on the head model at which the impact occurs.

11. A computer system configured to perform a method for prediction of head impact event mechanism, the method including:

    identifying a data set including measurements of linear and rotational acceleration of a head during an impact event;
    processing the linear acceleration thereby to predict an impact direction for the impact event;
    processing the dataset thereby to calculate a predicted moment arm associated with rotational movement described by the measurements of linear and angular accelerations;
    processing, in combination: (i) the predicted direction of the impact; and (ii) the predicted moment arm, thereby to determine a predicted impact location for the impact event.

12. A system according to claim 11 wherein the step of processing the linear and angular accelerations to calculate a moment arm required to produce a rotational movement described by the linear and angular accelerations is based on performed based on an assumption that the axis of rotation passes through the centre of gravity of the head.

13. A system according to claim 11 or claim 12, the method additionally including generating a graphic based on the predicted impact location for the impact event, wherein the graphic includes a representation of a human head, and an element graphically representing direction and location of impact.

14. A system according to claim 11, claim 12 or claim 14, the method additionally including estimation of a head orientation relative to a defined system of reference at one or more points in time during the impact event.

15. A system according to claim 14 wherein the estimation of a head orientation relative to the defined system of reference are used thereby to provide an animation of changes in head orientation during the impact event.

16. A system according to any one of claims 11 to 15 wherein the data set is derived from an instrumented mouthguard device.

17. A system according to any one of claims 11 to 16, the method additionally including:

    (i) rotating values for linear and angular accelerations to match a predefined system of reference;
    (ii) generating an array with spatial coordinates of a number of points on the surface of the head model;

(iii) using the coordinates of each point on the surface of the head model to generate vectors with origin at a tip of a moment arm vector;

(iv) calculating unit vectors for each vector generated at (iii);

(v) mathematically constructing a shape with centre at the centre of gravity of the head model;

(vi) defining two positions along a line of action of the force, being the tip of the momentum arm and the tip of the vector resulting from the sum of the moment arm and the linear acceleration vector;

(vii) using those two positions to generate the parametrized equations of the line of action of the force;

(viii) combining a shape equation for the shape and parametric equations for the line of action of the impact, thereby to calculate one or more intersections of the shape and the line of action, and thereby determine a point at which the impact enters the mathematically constructed shape;

(ix) constructing a vector from the tip of the moment arm to the point at which the impact enters the mathematically constructed shape, and normalizing that vector;

(x) identifying the combination of unit vector calculated at (iv) and vector calculated at (ix) having the smallest angle therebetween, and identifying an associated spatial coordinate on the head model for that unit vector, thereby to predict the spatial coordinate on the head model at which the impact occurs.

**FIG. 1**

# FIG. 2

Europäisches
Patentamt

European
Patent Office

Office européen
des brevets

# EUROPEAN SEARCH REPORT

Application Number

EP 22 19 9356

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2020/132212 A2 (CLEVELAND CLINIC FOUND [US]) 25 June 2020 (2020-06-25) * abstract; claims 1-55; figures 1-17 * * paragraphs [0015], [0054] – [0056], [0083], [0097] – [0114] * | 1-17 | INV. G01L5/00 A61B5/11 A63B69/00 A63B71/08 G01P15/08 G01P15/18 |
| A | WO 2021/179041 A1 (HITIQ LTD [AU]) 16 September 2021 (2021-09-16) * the whole document * | 1-17 | |
| A | US 2002/060633 A1 (CRISCO JOSEPH J [US] ET AL) 23 May 2002 (2002-05-23) * the whole document * | 1-17 | |
| A | US 2020/197785 A1 (GONZALES ANTHONY M [US] ET AL) 25 June 2020 (2020-06-25) * the whole document * | 1-17 | |

TECHNICAL FIELDS
SEARCHED      (IPC)

G01L
A63B
A61B
G01P

The present search report has been drawn up for all claims

| Place of search | Date of completion of the search | Examiner |
|---|---|---|
| The Hague | 20 February 2023 | Moscelli, Nicola |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 22 19 9356

This annex lists the patent family members relating to the patent documents cited in the above-mentioned European search report.
The members are as contained in the European Patent Office EDP file on
The European Patent Office is in no way liable for these particulars which are merely given for the purpose of information.

20-02-2023

| Patent document cited in search report | | Publication date | Patent family member(s) | | Publication date |
|---|---|---|---|---|---|
| WO 2020132212 | A2 | 25-06-2020 | AU | 2019404197 A1 | 05-08-2021 |
| | | | EP | 3899985 A2 | 27-10-2021 |
| | | | US | 2020312461 A1 | 01-10-2020 |
| | | | WO | 2020132212 A2 | 25-06-2020 |
| WO 2021179041 | A1 | 16-09-2021 | AU | 2021107528 A4 | 06-01-2022 |
| | | | AU | 2021233383 A1 | 27-10-2022 |
| | | | EP | 4117512 A1 | 18-01-2023 |
| | | | WO | 2021179041 A1 | 16-09-2021 |
| US 2002060633 | A1 | 23-05-2002 | US | 2002060633 A1 | 23-05-2002 |
| | | | US | 2005177335 A1 | 11-08-2005 |
| | | | US | 2014039355 A1 | 06-02-2014 |
| | | | US | 2017209092 A1 | 27-07-2017 |
| US 2020197785 | A1 | 25-06-2020 | AU | 2019401356 A1 | 17-06-2021 |
| | | | AU | 2022201324 A1 | 24-03-2022 |
| | | | CA | 3121194 A1 | 25-06-2020 |
| | | | EP | 3897888 A2 | 27-10-2021 |
| | | | EP | 4070864 A1 | 12-10-2022 |
| | | | JP | 2022514944 A | 16-02-2022 |
| | | | US | 2020196946 A1 | 25-06-2020 |
| | | | US | 2020196947 A1 | 25-06-2020 |
| | | | US | 2020197785 A1 | 25-06-2020 |
| | | | US | 2020375534 A1 | 03-12-2020 |
| | | | US | 2022346711 A1 | 03-11-2022 |
| | | | US | 2022361816 A1 | 17-11-2022 |
| | | | WO | 2020132548 A2 | 25-06-2020 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- AU 2020050096 W